# EUROPEAN PATENT APPLICATION

(11) **EP 1 118 677 A1**
(43) Date of publication of application: **25.07.2001**
(21) Application number: 00100808.5
(22) Date of filing: 17.01.2000
(51) Int. Cl.: C12Q 1/68

(54) **OLIGONUCLEOTIDES USEFUL IN IDENTIFYING FUNGICIDE RESISTANT PLANT PATHOGENIC FUNGI**

(71) Applicant: Novartis AG, 4002 Basel (CH)
(72) Inventor: The designation of the inventor has not yet been filed

(57) **Abstract**

The present invention provides unique oligonucleotides that are useful in identifying fungicide resistant plant pathogenic fungi , but especially pathogenic fungi that are resistant to fungicides which are targeted to the Qo center of the cytochrome b gene such as, for example, the so-called STAR fungicides. Particularly, the oligonucleotides according to the invention can be used to develop primers for a PCR-based analysis and differentiation between resistant and sensitive fungal pathotypes, and to monitor disease development in plant populations.

SEQUENCE LISTING

<110> Novartis AG

<120> Detection of fungicide resistant pathogens using the
      polymerase chain reaction

<130> Sierotzki

<140>
<141>

<160> 11

<170> PatentIn Ver. 2.1

<210> 1
<211> 26
<212> DNA
<213> Artificial Sequence

<220>
<223> Description of Artificial Sequence: Synthetic
      Oligonucleotide

<400> 1
ccttatggtc aaatgtcttt atgagc                                      26


<210> 2
<211> 26
<212> DNA
<213> Artificial Sequence

<220>
<223> Description of Artificial Sequence: Synthetic
      Oligonucleotide

<400> 2
ccttatggtc aaatgtcttt atgagg                                      26


<210> 3
<211> 25
<212> DNA
<213> Artificial Sequence

<220>
<223> Description of Artificial Sequence: Synthetic
      Oligonucleotide

<400> 3
ggcaaccttc agcttctata aatcc                                       25

<210> 4
<211> 18
<212> DNA
<213> Artificial Sequence

<220>
<223> Description of Artificial Sequence: Synthetic
      Oligonucleotide

<400> 4
gcagatgagc cactgggc                                                    18


<210> 5
<211> 18
<212> DNA
<213> Artificial Sequence

<220>
<223> Description of Artificial Sequence: Synthetic
      Oligonucleotide

<400> 5
gcagatgagc cactgggg                                                    18


<210> 6
<211> 23
<212> DNA
<213> Artificial Sequence

<220>
<223> Description of Artificial Sequence: Synthetic
      Oligonucleotide

<400> 6
cattcagagc ttggctaagt ccg                                              23


<210> 7
<211> 1140
<212> DNA
<213> Erysiphe graminis

<400> 7
tcattattag ctgtatcttt agttatacaa ataattacag gtgtaacttt agcaatgcat 60
tacaacccta gcgtattaga agcatttaac tcggtagagc atattatgcg ggatgttaat 120
aacggatgat taatacgtta catacactca aacacagctt cagctttctt tttcttagtt 180
tatttacata taggtagagg tttatattac ggatcatata gagcaccaag aacattagtt 240
tgaacaattg gtacagtaat attcatatta atgatcgtta ctgcattcct gggttatgta 300
ttgccatacg ggcagatgag ccactggggt gcaaccgtta tcactaacct aatgagcgct 360
ataccatgaa taggacaaga tattgtggag ttcctttgag gaggtttctc tgtaaataat 420
gcaacgttaa acagattctt tgctttacac tttgtcttgc cgttcgtttt agctgcttta 480
gctttaatgc acttaatagc acttcacgat agtgcaggat ctggtaatcc tttaggtgtt 540
tcaggtaatt acgatagatt acctatggct ccatacttct tatttaaaga tttaataact 600

atatttttat ttattattat tttaagtatg tttgtattct ttatgtcaaa tgttttaggg 660
gatagtgaga attatgtaat ggcaaatcca atgcaaactc cggctgctat tgtaccggag 720
tgatatcttc tgcccttcta tgcaatatta agatctatac ctaacaaatt attaggagtt 780
attgcaatgt ttagtgcaat tttagcttta ttggctatgc catttacaga tttaagtaga 840
agtagaggtc tacaattcaa acctttaaat aaagcagctt tctatatatt tataggtaat 900
tttgttatat taatggtttt aggtgcaaaa catgttgaat ctccttatat tgagttagga 960
caatttagta cacttttata tttttcacat tttttattaa tagtaccttt agttaatttt 1020
ttaaaaaaaa ctttaattgt attatcttgt agatctagct agaatattta gtggcatcgg 1080
acttagccaa gctctgaatg ccagtcgggt ctaaggccaa cttgtcaact gacgaagtct 1140

<210> 8
<211> 1140
<212> DNA
<213> Erysiphe graminis

<400> 8
tcattattag ctgtatcttt agttatacaa ataattacag gtgtaacttt agcaatgcat 60
tacaacccta gcgtattaga agcatttaac tcggtagagc atattatgcg ggatgttaat 120
aacggatgat taatacgtta catacactca aacacagctt cagctttctt tttcttagtt 180
tatttacata taggtagagg tttatattac ggctcatata gagcaccaag aacattagtt 240
tgaacaattg gtacagtaat attcatatta atgatcgtta ctgcattcct gggttatgta 300
ttgccatacg ggcagatgag ccactgggct gcaaccgtta tcactaacct aatgagcgct 360
ataccatgaa taggacaaga tattgtggag ttcctttgag gaggtttctc tgtaaataat 420
gcaacgttaa acagattctt tgctttacac tttgtcttgc cgttcgtttt agctgcttta 480
gctttaatgc acttaatagc acttcacgat agtgcaggat ctggtaatcc tttaggtgtt 540
tcaggtaatt acgatagatt acctatggct ccatacttct tatttaaaga tttaataact 600
atatttttat ttattattat tttaagtatg tttgtattct ttatgtcaaa tgttttaggg 660
gatagtgaga attatgtaat ggcaaatcca atgcaaactc cggctgctat tgtaccggag 720
tgatatcttc tgcccttcta tgcaatatta agatctatac ctaacaaatt attaggagtt 780
attgcaatgt ttagtgcaat tttagcttta ttggctatgc catttacaga tttaagtaga 840
agtagaggtc tacaattcaa acctttaaat aaagcagctt tctatatatt tataggtaat 900
tttgttatat taatggtttt aggtgcaaaa catgttgaat ctccttatat tgagttagga 960
caatttagta cacttttata tttttcacat tttttattaa tagtaccttt agttaatttt 1020
ttagaaaata ctttaattgt attatcttgt agatctagct agaatattta gtggcatcgg 1080
acttagccaa gctctgaatg ccagtcgggt ctaaggccaa cttgtcaact gacgaagtct 1140

<210> 9
<211> 1140
<212> DNA
<213> Erysiphe graminis

<400> 9
tcattattag ctgtatcttt agttatacaa ataattacag gtgtaacttt agcaatgcat 60
tacaacccta gcgtattaga agcatttaac tcggtagagc atattatgcg ggatgttaat 120
aacggatgat taatacgtta catacactca aacacagctt cagctttctt tttcttagtt 180
tatttacata taggtagagg tttatattac ggatcatata aagcaccaag aacattagtt 240
tgaacaattg gtacagtaat attcatatta atgatcgtta ctgcattcct gggttatgta 300
ttgccatacg ggcagatgag ccactgggct gcaaccgtta tcactaacct aatgagcgct 360
ataccatgaa taggacaaga tattgtggag ttcctttgag gaggtttctc tgtaaataat 420
gcaacgttaa acagattctt tgctttacac tttgtcttgc cgttcgtttt agctgcttta 480
gctttaatgc acttaatagc acttcacgat agtgcaggat ctggtaatcc tttaggtgtt 540
tcaggtaatt acgatagatt acctatggct ccatacttct tatttaaaga tttaataact 600
atatttttat ttattattat tttaagtatg tttgtattct ttatgtcaaa tgttttaggg 660

gatagtgaga attatgtaat ggcaaatcca atgcaaactc cggctgctat tgtaccggag 720
tgatatcttc tgcccttcta tgcaatatta agatctatac ctaacaaatt attaggagtt 780
attgcaatgt ttagtgcaat tttagcttta ttggctatgc catttacaga tttaagtaga 840
agtagaggtc tacaattcaa acctttaaat aaagcagctt tctatatatt tataggtaat 900
tttgttatat taatggtttt aggtgcaaaa catgttgaat ctccttatat tgagttagga 960
caatttagta cacttttata tttttcacat tttttattaa tagtaccttt agttaatttt 1020
ttagaaaata ctttaattgt attatcttgt agatctagct agaatattta gtggcatcgg 1080
acttagccaa gctctgaatg ccagtcgggt ctaaggccaa cttgtcaact gacgaagtct 1140

<210> 10
<211> 2071
<212> DNA
<213> Mycosphaerella fijiensis

<400> 10
tacaatccta gtgtattaga agcatttaat tcagtagaac acataatgag agatgtaaat 60
aatggatgat taatacgtta tttacactca aatactgcct cagctttttt ctttttagtg 120
tacttacacg taggaagagg tttatactat ggatcatata aagcacctag aacattagta 180
tgagttatag gtactattat attagttcta atgatggcaa ctgccttttt agggtatgtt 240
ttaccttatg gtcaaatgtc tttatgagca gctacagtta taactaattt aatgagcgct 300
ataccctgag taggacaaga tatagttgag tttatttgag gaggtttata tatagatgaa 360
ccacattacg gcgacgtact gttaaaaatc ctgcttaatg ctggaacatc ccccagttta 420
ggatttgcat acgacttttt cttaatattt atattaatta ttggcgtaaa aattgcaatg 480
acatggggac aatcagcagg ggtgagaagt attcatactt cagaagcctc tcagagacta 540
catgcaggag atctcactta cgcttattta gtaggattat ttgaaggaga tggtttcttt 600
tacattacaa aaaaaggtaa atatgtaaca tacgaactag gtatagaatt atctattaaa 660
gacgtacaat taatttataa aattaaagct ttattaggta taggagttgt aaggtttaga 720
acaataaatg agtcagaaat ggttgcccta ataattagag atataaacca gttaaaaagc 780
tttatattac ctatatttga caaatatcct atgttttctg ataaacaata tgactattta 840
agatttagaa atgctttagt atcaggtatt atatatttta aaggatttac ctgaatacac 900
taaaagtaat ctgcctttaa attctataga atctatttta aataaatctt atttttccgc 960
ttgattagta ggatttatag aagctgaagg ttgcctttag tatatataaa ttgaaaaaaa 1020
acgataacta tttaatagct agtttttata ttgctcagaa aaatggtgat attgtaataa 1080
aagctatttc tacatattta tcctttacta ctggcattta tttagacaaa tcttattgtt 1140
ctacattaaa agttacaggt ataagatcaa tagaaaatac tattaacttt ttagataaag 1200
cacctgtaaa actaatgggt aataaaagat tgcaatattt attatggata aaacaattac 1260
gtacaatacc tagatattca gaaaatataa aaatcccttc aatataccgc taataaaaat 1320
gagatcatga tatagtccga tcaataaaga aatttattgg gtgtagtgag aatatttaat 1380
ttacattaaa tatggcgact accaacacaa atgctctgtt aacaatgcta cattaaatag 1440
attctttgct ttacattttg tttttccatt cgtattagct gcattagcat taatgcattt 1500
aattgcatta cacgatagtg caggttcagg taatccttta ggagtatcag gaaactatga 1560
cagacttcct tttgctccat acttcatctt taaggatctg ataactatct ttttatttat 1620
agtagtgtta tctatattcg tatttttcat gcctaatgtt ttaggtgaca gtgaaaacta 1680
tgtaatggct aaccctatgc aaacaccccc agctattgtg cctgagtgat atcttttacc 1740
tttctatgct atattaagat ctatacctaa caaattatta ggtgttattg caatgttttc 1800
tgcaatctta ataataatga ttatgccatt tacagattta ggtagaagta gaggattaca 1860
attcagacct ttaagtaaaa tagcattttt tgttttcgta gcaaattttt tagttttaat 1920
gcaantagga gctaaacatg ttgaatcccc atatatagaa ttcggtcaaa taagaactgt 1980
tctatatttt tcacacttct taatcatagt tcctttactt agcatattag aaaatacttt 2040
aatagattta caccttaata acccaaagta a 2071

<210> 11
<211> 2071

<212> DNA
<213> Mycosphaerella fijiensis

<400> 11
tacaatccta gtgtattaga agcatttaat tcagtagaac acataatgag agatgtaaat 60
aatggatgat taatacgtta tttacactca aatactgcct cagctttttt ctttttagtg 120
tacttacacg taggaagagg tttatactat ggatcatata aagcacctag aacattagta 180
tgagttatag gtactattat attagttcta atgatggcaa ctgccttttt agggtatgtt 240
ttaccttatg gtcaaatgtc tttatgagga gctacagtta taactaattt aatgagcgct 300
ataccctgag taggacaaga tatagttgag tttatttgag gaggtttata tatagatgaa 360
ccacattacg gcgacgtact gttaaaaatc ctgcttaatg ctggaacatc ccccagttta 420
ggatttgcat acgacttttt cttaatattt atattaatta ttggcgtaaa aattgcaatg 480
acatggggac aatcagcagg ggtgagaagt attcatactt cagaagcctc tcagagacta 540
catgcaggag atctcactta cgcttattta gtaggattat ttgaaggaga tggtttcttt 600
tacattacaa aaaaaggtaa atatgtaaca tacgaactag gtatagaatt atctattaaa 660
gacgtacaat taatttataa aattaaagct ttattaggta taggagttgt aaggtttaga 720
acaataaatg agtcagaaat ggttgcccta ataattagag atataaacca gttaaaaagc 780
tttatattac ctatatttga caaatatcct atgttttctg ataaacaata tgactattta 840
agatttagaa atgctttagt atcaggtatt atatatttta aaggatttac ctgaatacac 900
taaaagtaat ctgcctttaa attctataga atctatttta aataaatctt atttttccgc 960
ttgattagta ggatttatag aagctgaagg ttgcctttag tatatataaa ttgaaaaaaa 1020
acgataacta tttaatagct agtttatata ttgctcagaa aaatggtgat attataataa 1080
aagctatttc tacatattta tcctttacta ctggcattta tttagacaaa tcttattgtt 1140
ctacattaaa agttacaggt ataagatcaa tagaaaatac tattaacttt ttagataaag 1200
cacctgtaaa actaatgggt aataaaagat tgcaatattt attatggata aaacaattac 1260
gtacaatacc tagatattca gaaaatataa aaatcccttc aatataccgc taataaaaat 1320
gagatcatga tatagtccga tcaataaaga aatttattgg gtgtagtgag aatatttaat 1380
ttacattaaa tatggcgact accaacacaa atgctctgtt aacaatgcta cattaaatag 1440
attctttgct ttacattttg tttttccatt cgtattagct gcattagcat taatgcattt 1500
aattgcatta cacgatagtg caggttcagg taatccttta ggagtatcag gaaactatga 1560
cagacttcct tttgctccat acttcatctt taaggatctg ataactatct tttatattat 1620
agtagtgtta tctatattcg tatttttcat gcctaatgtt ttaggtgaca gtgaaaacta 1680
tgtaatggct aaccctatgc aaacaccccc agctattgtg cctgagtgat atcttttacc 1740
tttctatgct atattaagat ctatacctaa caaattatta ggtgttattg caatgttttc 1800
tgcaatctta ataataatga ttatgccatt tacagattta ggtagaagta gaggattaca 1860
attcagacct ttaagtaaaa tagcattttt tgttttcgta gcaaattttt tagttttaat 1920
gcaantagga gctaaacatg ttgaatcccc atatatagaa ttcggtcaaa taagaactgt 1980
tctatatttt tcacacttct taatcatagt tcctttactt agcatattag aaaatacttt 2040
aatagattta caccttaata acccaaagta a 2071

## Description

The present invention provides unique oligonucleotides that are useful in identifying fungicide resistant plant pathogenic fungi , but especially pathogenic fungi that are resistant to fungicides which are targeted to the Qo center of the cytochrome b gene such as, for example, the so-called STAR fungicides. Particularly, the oligonucleotides according to the invention can be used to develop primers for a PCR-based analysis and differentiation between resistant and sensitive fungal pathotypes. and to monitor disease development in plant populations.

Diseases in plants cause considerable crop loss from year to year resulting both in economic deprivation to farmers and, in many parts of the world, to shortfalls in the nutritional provision for local populations. The widespread use of fungicides has provided considerable security against plant pathogen attack. However, despite $1 billion worth of expenditure on fungicides, worldwide crop losses amounted to approximately 10% of crop value in 1981. One of the reasons for these crop losses is emergence of fungicide resistant pathotypes in an increasing number of pathogen populations.

There are several documented cases of the evolution of fungal strains that are resistant to particular fungicides. As early as 1981, 24% of the powdery mildew populations from spring barley and 53% from winter barley showed considerable variation in response to the fungicide triadimenol and the distribution of these populations varied between varieties, with the most susceptible variety also giving the highest incidence of less susceptible types. Similar variation in the sensitivity of fungi to fungicides has been documented for wheat mildew (also to triadimenol), *Botrytis* (to benomyl), *Pyrenophora* (to organomercury), *Pseudocercosporella* (to MBC-type fungicides) and *Mycosphaerella fijiensis* to triazoles.

Respiration inhibitors at the Qo center of the cytochrome b gene such as, for example STAR (Strobilurin Type Action and Resistance) fungicides are introduced recently to the market as efficient and specific fungicides, such as kresoxim methyl, azoxystrobin, trifloxystrobin and famoxadone. Since these inhibitors specifically affect the function of the cytochrome bc1 enzyme complex, a large amount of DNA sequence data and enzyme structure information is available. Moreover, the molecular basis of strobilurin resistant mutants and strobilurin-producing fungi has been elucidated: In total, eleven point-mutations are known to confer resistance to centre Qo inhibitors being located in two regions of the cytochrome b gene (Aa 127-147 and Aa 275-296); it is suggested that these regions come close to each other in the folded protein and are important for ligand binding. Only one case of an upregulation in respiration rate is described as supporting resistance. Recently, a report has been published claiming resistance of *Erysiphe graminis* DC. f. sp. *tritici* Em.Marchal to strobilurin fungicides. It is argued that insufficient treatments or resistant subpopulation may have caused the control failures, and that resistant isolate may not be controlled by increased dosages and will not disappear during winter. Furthermore, it is suspected that all "strobilurins" are affected similarly because of their cross resistance behaviour.

It is expected that fungicide resistant forms of fungal pathogens will continue to emerge in the pathogen populations in response to the introduction of fungicides. It is therefore necessary to monitor the frequency of resistant isolates in particular pathogen populations. There is, therefore, a desire for a method which allows to screen for fungicide resistant pathogens in an early stage of the development of fungicide resistance.
The invention, which is described in the following in detail provides such a method for detecting and distinguishing resistant isolates of fungal pathogens in a species-specific manner.

For clarity, certain terms used in the specification are defined and presented as follows:
Allele: alternative form of a gene, differing in at least a single base change.
Allele-specific primer: An allele specific primer is an oligonucleotide recognising the nucleotide change(s) between two alleles. In the context of the invention, an alllele specific primer is understood to comprise oligonucleotides that differ from each other, for example, in one or more nucleotides at their terminal 3'-end. Under the primer annealing tempeature and PCR conditions (stringent, selective conditions), these primers only direct amplification on their complementary allele. Such primers are used in allele-specific amplification - polymerase chain reaction analysis to examine nucleotide sequences differing in up to a single base in a codon resulting in an amino acid substitution in a particular gene. This substituion leads to an change in the phenotype of the particular individual.
Gene: refers to a coding sequence and associated regulatory sequences wherein the coding sequence is transcribed into RNA such as mRNA, rRNA, tRNA, snRNA, sense RNA or antisense RNA. Examples of regulatory sequences are promoter sequences, 5' and 3' untranslated sequences and termination sequences. Further elements that may be present are, for example, introns.
Identity: The percentage of sequence identity is determined using computer programs that are based on dynamic programming algorithms. Computer programs that are preferred within the scope of the present invention include the BLAST (Basic Local Alignment Search Tool) search programs designed to explore all of the available sequence databases regardless of whether the query is protein or DNA. Version BLAST 2.0 (Gapped BLAST) of this search tool has been made publicly available on the Internet (currently http://www.ncbi.nlm.nih.gov/BLAST/). It uses a heuristic algorithm which seeks local as opposed to global alignments and is therefore able to detect relationships among sequences which share only isolated regions. The scores assigned in a BLAST search have a well-defined statistical interpretation. Said programs are preferably run with optional parameters set to the default values
Plant: refers to any plant, particularly to seed plants
Primer: Short preexisting polynucleotide chain to which new deoxyribonucleotides can be added by DNA polymerase.
Polymerase chain reaction (PCR): A method for amplifying a DNA base sequence using a heatstable polymerase and two oligonucleotide primers, one complementary to the (+) strand (reverse) at one end of the sequence to be amplified and the other complementary to the (-) strand (foreward) at the other end. Because the newly synthesized DNA strands can subsequently serve as additional templates for the same primer sequences, successive rounds of primer annealing, strand elongation, and dissociation produce rapid and highly specific amplification of the desired sequence. PCR also can be used to detect the existence of the defined sequence in a DNA sample.
Recombinant: procedures used to join together DNA sequences as described, for example , in Sambrook et al., 1989 Cold SpringHarbor, NY: Cold Spring Harbor Laboratory Press.
Species specific primer: The species specific primer is an oligonucleotide recognising differnces in the nucleotide sequence between two species. The species specific primers differ from each other in the nucleotide sequence. Under the primer annealing tempeature and PCR conditions (stringent, selective conditions), these primers only direct amplification on their complementary nucleotide sequence. Such primers are used in species-specific amplification -polymerase chain reaction analysis to differentiate between species.

The present invention is drawn to a method of identifying fungicide resistant pathotypes of plant pathogenic fungi, but especially of pathogenic fungi that are resistant to fungicides which are targeted to the Qo center of the cytochrome b gene such as, for example, the so-called STAR fungicides.

The invention provides DNA oligonucleotides that are suitable to detect mutations in a fungicide target gene such as, for example, the cytochrome b gene, of a pathogen isolate which mutation confers resistance to said fungicide. In a preferred embodiment, the invention provides DNA oligonucleotides that are capable to detect mutations in the cytochrome b gene of a pathogen isolate of the fungal pathogens *Erysiphe graminis* DC. f. sp. *tritici and Mycosphaerella fijiensis,* respectively.

Such oligonucleotides can be suitably used in the method of the invention to develope primers for use in polymerase chain reaction (PCR)-based diagnostic assays.

In a specific embodiment of the invention, a first set of primers is designed which is allele-specific and thus capable to detect and distinguish the different alleles of resistant and sensitive fungal strains, respectively. In case of the cytochrome b gene said alleles are preferably distinguished by at least one point mutation, preferably within a region that is important for ligand binding such as, for example the region corresponding to amino acid position 127-147 and 275-296 respectively. Especially preferred within the invention is a region corresponding to amino acid position 143.

A second set of primers is designed, which is capable of recognizing DNA sequences, which are specific for the fungal species to be detected and can thus be used, in combination with the allele-specific primer, to identify the presence or absence of specific pathotypes (sensitive or resistant) in host plant material.

The two sets of primers can be used in a PCR reaction to amplify allele- and species-specific fragments of a fungal pathogen.

The invention further provides a detection method that is especially suitable for detecting fungal pathogens which are resistant to specific fungicides.

In addition, a method is described for extracting DNA from plant tissue for high throughput PCR analysis.

Kits useful in the practice of the invention are also provided. The kits find particular use in the identification of the fungicide resistant pathogens *Erysiphe graminis* DC. f. sp. *tritici and Mycosphaerella fijiensis,* respectively.

This invention can further be used to provide detailed information on the development and spread of specific pathogen races which are resistant to fungicides over extended geographical areas.

The present invention provides unique oligonucleotides that are useful in identifying fungicide resistant plant pathogenic fungi , but especially pathogenic fungi that are resistant to fungicides which are targeted to the Qo center of the cytochrome b gene such as, for example, the so-called STAR fungicides. Particularly, the oligonucleotides according to the invention can be used to develop primers for a PCR-based analysis and differentiation between resistant and sensitive fungal pathotypes.
The oligonucleotides of the invention include oligonucleotides which correspond to suitable parts of the cytochrome b gene of particular fungal pathogens which are different in the resistant and sensitive pathotype and can thus be suitable used for identifying the resistant pathotype. Said differences are based on mutations, preferably point mutations, resulting in at least one single base exchange and leading to fungicide resistance.
Preferred are those regions or parts of the cytochrome b gene which are important for ligand binding such as, for example the regions comprising amino acid position 127-147 and 275-296, respectively.

Based on said oligonucleotides primers can be developed which are suitable for detecting resistant and/or sensitive pathotypes of fungal pathogens. Alternatively, said oligonucleotides can be used as such in hybridization-type reactions know in the art. Furthermore, oligonucleotides from different pathogen species or primers developed therefrom can be used to identify those pathogen species, provided that said sequences vary between the different species such that they anneal diffentially to templates under particular hybridization and PCR conditions, respectively.

The length of the primers may vary dependent on the annealing temperature in the PCR reaction, which preferably is from 35°C to 80°C, more preferably from 50°C to 75°C and most preferably from 60°C to 72°C . The preferred size of the oligonucletide primers to be used within the invention is from 15 to 40 nucleotides, more preferably from 18 to 30 nucleotides, and most preferably from 18 to between 23 and 26 oligonucleotides.

Examples of such oligonucleotide DNA sequences are disclosed in SEQ ID NOs:1-6, which are obtained from the cytochrome b gene region of *M. fijiensis and E. graminis* f. sp. *Tritici,* respectively.
*M. fijiensis*: SEQ ID NO:1: CCTTATGGTCAAATGTCTTTATGAGC resistant SEQ ID NO:2: CCTTATGGTCAAATGTCTTTATGAGG sensitive 
SEQ ID NO:3: GGCAACCTTCAGCTTCTATAAATCC specific for M. fijiensis (resistant and
sensitive)
and *E. graminis* f. sp. *Tritici, respectively:*

SEQ ID NO:4: GCAGATGAGCCACTGGGC resistant

SEQ ID NO:5: GCAGATGAGCCACTGGGG sensitive

SEQ ID NO:6: CATTCAGAGCTTGGCTAAGTCCG specific for E. graminis f. sp. tritici
(resistant and sensitive)

Also encompassed by the invention is an oligonucleotide primer for use in amplification-based detection of a fungal pathogen, wherein said primer has sequence identity with at least about 5-10 nucleotides of the sequence selected from the group consisting of SEQ ID NO:SEQ ID NO: 1, SEQ ID NO:SEQ ID NO: 2, SEQ ID NO:SEQ ID NO: 3 SEQ ID NO:SEQ ID NO: 4 SEQ ID NO:SEQ ID NO: 5 SEQ ID NO:SEQ ID NO: 6, preferably.

In order to be able to distinguish between sensitive and resistant isolates wherein the resistance phenotype is due to a point-mutation in the gene encoding a target enzyme of a STAR fungicide, but especially the gene encoding a cytochrom bc₁ enzyme complex, primers are constructed such that they anneal differently to DNA of sensitive or resistant isolates, at appropriate amplification conditions (allele-specific primers). Preferred are primers that are capable to differentiate between the various allelic genes based on a point mutation within a region that is important for ligand binding such as, for example the region corresponding to amino acid position 127-147 and 275-296 respectively. Especially preferred within the invention is a region corresponding to amino acid position 143 in the cytochrom b protein.

Especially preferred within the scope of the invention are primers that are capable of distinguishing between sensitive and resistant isolates of *Erysiphe graminis* and *Mycosphaerella fijiensis,* respectively. The allele-specific primers are designed such that the sequence difference distinguishing between sensitive and resistant isolates is preferably located at the 3'- end of the respective primer. In case of a point mutation the primer perferably differentiate in at least one nucleotide, which nucleotide is preferably located in a region at the 3'-primer end comprising between 1 to 5, preferably between 1 to 3 and most preferably 1 nucleotide.
In oder to be able to distinguish between different species, species-specific primers are designed that anneal to regions that are unique to the particular species and in a suitable distance for PCR amplification, which is about 100 to about 10'000, preferably about 100 to about 4000 and most preferably about 100 to about 2000 base pairs apart from the region annealing with the allele-specific primers.
Encompassed by the invention is a method of constructing a primer for the detection of resistant isolates of a plant fungal pathogen, preferably a pathogen that is resistant to a fungicide targeting the Qo center of the cytochrome b gene such as, for example, the STAR fungicides, but especially a pathogen selected from the group consisting of *M. fijiensis or* a *E. graminis* f. sp. *tritici, respectively* In case of a point mutation the primer is capable to differentiate up to one nucleotide, which nucleotide is preferably located in a region at the 3'- end of the primer comprising between 1 to 5, preferably between 1 to 3 and most preferably 1 nucleotide. Especially preferred is a primer that recognizes *a point mutation leading to a single base exchange in the resistant allele in a triplet corresponding to* amino acid position 143 of the cytochrom b gene and wherein said changed base is the last base at the 3'- end of the primer.

Further comprised is a method of constructing a primer for distingushing different species or even different pathotypes within the same species or genus, wherein said second primer (species specific primers) anneals to DNA sequences which are identical within a given species and differential among species in about 100 to about 10'000, preferably about 100 to about 4000 and most preferably about 100 to about 2000 base pairs apart from the region annealing with the allele-specific primers.

The oligonucletides according to the invention can be derived from sequences SEQ ID NO:SEQ ID NO: 7 to 11 disclosing a sequence of the cytochrome b gene of a sensitive (SEQ ID NO: 11) and a STAR-resistant (SEQ ID NO: 10) isolate of *Mycosphaerella fijiensis* and the sequence of the cytochrome b gene of a sensitive (SEQ ID NO:7) and two STAR-resistant (SEQ ID NO: No:8 and 9) isolates of *Erysiphe graminis.*
The sequences obtained are 2040 and 1140 base pairs long for *M. fijiensis* and *E. graminis,* respectively. The cytochrome b gene of *M. fijiensis* contains a 1063 long intron from position 349 to 1412. The coding regions are 1006 and 1060 bp long for *M. fijiensis* and *E. graminis,* respectively. Comparison of the sequence of the cytochrome b gene of a sensitive (SEQ ID
NO: 11) and a STAR-resistant (SEQ ID NO: 10) isolate of *Mycosphaerella fijiensis* reveale a difference at nucleotide position 269 (change from G to C) leading to a change of the deduced amino acid sequence from glycine to alanine at position 143. Two additional differences are observed but are not important for the amino acid sequence, because they are in the intron. Comparison of the sequence of the cytochrome b gene of a sensitive (SEQ ID NO:7) and two STAR- (SEQ ID No:8 and 9) isolates of *Erysiphe graminis* reveale a difference at nucleotide position 350 (change from G to C) leading to a change of the deduced amino acid sequence from glycine to alanine at position 143. Additional differences are observed but are less important, because they are in parts of the gene not contributing to the binding of the inhibitors. The stop codon is at position 1060.
The above nucleotide sequences as represented by SEQ ID NO: NOs 7 to 11 are also part of the present invention.
Further encompassed by the invention is a pair of oligonucleotide primers for use in the amplification-based differentiation between sensitive and resistant isolates in a species-specific manner. These primer pairs are characterized in that they are capable of recognising sensitive or resistant isolates, as well as the specific species (resistant and sensitive).
Among a given pathogen species the allele-specific primer perferably differentiate in the nucleotides at the 3'- end of the respective primer. In case of a point mutation the primer perferably differentiate in at least one nucleotide, which nucleotide is preferably located in a region at the 3'-primer end comprising between 1 to 5, preferably between 1 to 3 and most preferably 1 nucleotide.
Whereas the second primer (species specific primers) anneals to DNA sequences which are identical within a given species and differential among species perferably in a region which is about 100 to about 10'000, preferably about 100 to about 4000 and most preferably about 100 to about 2000 base pairs apart from the region annealing with the allele-specific primers.

Encompassed by and preferred within the scope of the invention are the following specific primer pairs:
Primer pair recognising sensitive isolates of *M. fijiensis*
   SEQ ID NO: 2 and SEQ ID NO: 3
Primer pair recognising resistant isolates of *M. fijiensis*
   SEQ ID NO: 1 and SEQ ID NO: 3
Primer pair recognising sensitive isolates of *E. graminis* f. sp. *tritici*
   SEQ ID NO: 5 and SEQ ID NO: 6
Primer pair recognising resistant isolates of *E. graminis* f. sp. *tritici*
   SEQ ID NO: 4 and SEQ ID NO: 6.

Encompassed by the invention is an oligonucleotide primer for use in amplification-based detection of a fungal pathogen, wherein said primer has sequence identity with at least about 5-10 nucleotides of the sequence selected from the group consisting of SEQ ID NO: 1, SEQ ID NO: 2, SEQ ID NO: 3 SEQ ID NO: 4 SEQ ID NO: 5 SEQ ID NO: 6, which 5-10 nucleotides are preferably located at the 3'- end of the respective primer.

Encompassed by the invention is a method for the detection of resistant isolates of a plant fungal pathogen, preferably a pathogen that is resistant to a fungicide targeting the Qo center of the cytochrome b gene such as, for example, the STAR fungicides, but especially a pathogen selected from the group consisting of *M. fijiensis or a E. graminis f.* sp. *tritici* comprising the steps of:
(a) isolating DNA from a plant, preferably a plant leaf, infected with a pathogen;
(b) subjecting said DNA to polymerase chain reaction amplification using
   (i) an allele-specific primer recognising DNA of resistant isolates in combination with
   (ii) a species-specific primer,
   wherein, in case of a point mutation, the allele-specific primer differentiate in at least one nucleotide at the 3'- end of the respective primer, which nucleotide is preferably located in a region at the 3'- end comprising between 1 to 5, preferably between 1 to 3 and most preferably 1 nucleotide
   and the species specific primer anneals to DNA sequences which are identical within a given species and differential among species
   perferably in a region which is about 100 to about 10'000, preferably about 100 to about 4000 and most preferably about 100 to about 2000 base pairs apart from the region annealing with the allele-specific primer, and
(c) detecting said fungal pathogen by visualizing the product or products of said polymerase chain reaction amplification.

Further encompassed by the invention is a method for distinguishing between resistant and sensitive isolates of a plant fungal pathogen, preferably a pathogen that is resistant to a fungicide targeting the Qo center of the cytochrome b gene such as, for example, the STAR fungicides, but especially a pathogen selected from the group of *M. fijiensis or* a *E. graminis* f. sp. *tritici* comprising the steps of:
(a) isolating DNA from a plant, preferably a plant leaf, infected with a pathogen;
(b) subjecting said DNA to polymerase chain reaction amplification using
   (I) an allele-specific primer recognising DNA of sensitive isolates in combination with a species-specific primer,
   (ii) an allele-specific primer recognising DNA of resistant isolates in combination with a species-specific primer,
   wherein, in case of a point mutation, the allele-specific primers differentiate in at least one nucleotide at the 3'- end of the respective primer, which nucleotide is preferably located in a region at the 3'-end comprising between 1 to 5, preferably between 1 to 3 and most preferably 1 nucleotide
   and the species specific primer anneals to DNA sequences which are identical within a given species and differential among species perferably in a region which is about 100 to about 10'000, preferably about 100 to about 4000 and most preferably about 100 to about 2000 base pairs apart from the region annealing with the allele-specific primer, and
(c) detecting said fungal pathogen by visualizing the product or products of said polymerase chain reaction amplification.

Further encompassed by the invention is a method for the detection of the frequency of resistant isolates of a plant fungal pathogen, preferably a pathogen that is resistant to a fungicide targeting the Qo center of the cytochrome b gene such as, for example, the STAR fungicides, but especially a pathogen selected from the group consisting of *M. fijiensis or* a *E. graminis* f. sp. *tritici* comprising the steps of:
(a) isolating DNA from a plant, preferably a plant leaf, infected with a pathogen;
(b) subjecting said DNA to polymerase chain reaction amplification using
   (I) an allele-specific primer recognising DNA of sensitive isolates in combination with a species-specific primer,
   (ii) an allele-specific primer recognising DNA of resistant isolates in combination with a species-specific primer,
   wherein, in case of a point mutation, the allele-specific primers differentiate in at least one nucleotide at the 3'- end of the respective primer, which nucleotide is preferably located in a region at the 3'-end comprising between 1 to 5, preferably between 1 to 3 and most preferably 1 nucleotide
   and the species specific primer anneals to DNA sequences which are identical within a given species and differential among species perferably in a region which is about 100 to about 10'000, preferably about 100 to about 4000 and most preferably about 100 to about 2000 base pairs apart from the region annealing with the allele-specific primer and
(c) detecting said fungal pathogen by visualizing the product or products of said polymerase chain reaction amplification and.
(d) quantifiying the PCR products and calculating the ratio.

Preferred is the method according to the invention, wherein said fungal pathogen is from the group consisting of *Erysiphe graminis* DC. f. sp. *Tritici and Mycosphaerella fijiensis.* Further preferred is the method according to the invention, wherein the allele-specific primer recognising DNA of sensitive isolates is the primer of SEQ ID No: 5, the allele-specific primer recognising DNA of resistant isolates is SEQ ID 4 and the species-specific primer specific for *Erysiphe graminis* DC. f. sp. *tritici* is the primer of SEQ ID NO: 6. Preferred is the method according to the invention, wherein the allele-specific primer recognising DNA of sensitive isolates is the primer of SEQ ID NO: 2, the allele-specific primer recognising DNA of resistant isolates is SEQ ID NO: 1 and the species-specific primer specific for *Mycosphaerella fijiensis* is the primer of SEQ ID NO: 3.

The methods and conditions employed in in PCR-based detection assays are well known in the art. For example, see U.S. Patent Nos. 4,683,195 and 4,683,202, as well as Schlesser *et al.* (1991) *Applied and Environ. Microbiol.* 57:553-556. See also, Nazar *et al.* (1991; *Physiol. and Molec. Plant Pathol*. 39: 1-11), which used PCR amplification to exploit differences in the ITS regions of *Verticillium albo-atrum* and *Verticillium dahliae* and therefore distinguish between the two species; and Johanson and Jeger (1993; *Mycol. Res.* 97: 670-674), who used similar techniques to distinguish the banana pathogens *Mycosphaerella fijiensis* and *Mycospharella musicola.*

The oligonucleotide DNA sequences of the invention can be cloned from fungal pathogens by methods known in the art. In general, the methods for the isolation of DNA from fungal isolates are described in: Raeder & Broda (1985) *Letters in Applied Microbiology 2*:17-20; Lee *et al.* (1990) *Fungal Genetics Newsletter 35*:23-24; and Lee and Taylor (1990) In: *PCR Protocols: A Guide to Methods and Applications,* Innes *et al.* (Eds.); pages 282-287.

The oligonucleotide sequences are determined and the sequences are compared within each pathogen group to locate divergences that might be useful to test in PCR to distinguish the different species and/or strains. Templates used for PCR-amplification testing are firstly purified pathogen DNA, and subsequently DNA isolated from infected host plant tissue. Thus, it is possible to identify pairs of primers that are diagnostic, *i.e.* that identified one particular pathogen species or strain but not another species or strain of the same pathogen and primers that identified resistant pathogen species.

Preferred primer combinations are able to distinguish between the different resistant or sensitve species or strains in infected host tissue, *i.e.* host tissue that has previously been infected with a specific pathogen species or strain. The primers of the invention are designed based on sequence differences among the fungal oligonucleotide regions and among sensitive and resistant fungal oligonucleotide regions. A minimum of one base pair difference between sequences can permit design of a discriminatory primer. Primers designed to a specific fungal DNA's oligonucleotide region can be used in combination with a primer made to a conserved sequence region within the ribosomal DNA's coding region to amplify species-specific PCR fragments. In general, primers should have a theoretical melting temperature between about 60 to about 70 degree °C to achieve good sensitivity and should be void of significant secondary structure and 3' overlaps between primer combinations. Primers are generally about 15-40 nucleotide bases, preferred 18-30, more preferred 18-28 nucleotide bases long.

The present invention lends itself readily to the preparation of "kits" containing the elements necessary to carry out the process. Such a kit may comprise a carrier being compartmentalized to receive in close confinement therein one or more container, such as tubes or vials. One of the containers may contain unlabeled or detectably labeled DNA primers. The labeled DNA primers may be present in lyophilized form or in an appropriate buffer as necessary. One or more containers may contain one or more enzymes or reagents to be utilized in PCR reactions. These enzymes may be present by themselves or in mixtures, in lyophilized form or in appropriate buffers.

Finally, the kit may contain all of the additional elements necessary to carry out the technique of the invention, such as buffers, extraction reagents, enzymes, pipettes, plates, nucleic acids, nucleoside triphosphates, filter paper, gel materials, transfer materials, autoradiography supplies, and the like.

### Description of the sequence listing

- **SEQ ID NO:1**: Primers used for the particular pathogen for *M. fijiensis* resistant
- **SEQ ID NO:2**: Primers used for the particular pathogen for *M. fijiensis* sensitive
- **SEQ ID NO:3**: Primers used for the particular pathogen for *M. fijiensis* resistant and sensitive
- **SEQ ID NO:4**: Primers used for the particular pathogen are *E. graminis* f. sp. *tritici* resistant
- **SEQ ID NO:5**: Primers used for the particular pathogen are *E. graminis* f. sp. *tritici* sensitive
- **SEQ ID NO:6**: Primers used for the particular pathogen are *E. graminis* f. sp. *tritici* resistant and sensitive
- **SEQ ID NO:7**: Cytochrome b gene of Erysiphe. *graminis* f*.* sp. *tritici* sensitive to fungicide
- **SEQ ID NO:8**: Cytochrome b gene of Erysiphe-. *graminis f.* sp. *tritici* resistant to fungicide
- **SEQ ID NO:9**: Cytochrome b gene of Erysiphe-. *graminis* f. sp. *tritici* resistant to fungicide
- **SEQ ID NO:10**: Cytochrome b gene of Mycosphaelella *fijiensis* resistant to fungicide
- **SEQ ID NO:11**: Cytochrome b gene of Mycosphaelella *fijiensis* sensitive to fungicide

The examples below show typical experimental protocols that can be used in the isolation of oligonucleotide sequences, the selection of suitable primer sequences, the testing of primers for selective and diagnostic efficacy, and the use of such primers for disease and fungal isolate detection. Such examples are provided by way of illustration and not by way of limitation.

### Examples

Standard recombinant DNA and molecular cloning techniques used here are well known in the art and are described by J. Sambrook, E. F. Fritsch and T. Maniatis, Molecular Cloning: A Laboratory manual, Cold Spring Harbor laboratory, Cold Spring Harbor, NY (1989) and by Ausubel, F.M. *et al.,* Current Protocols in Molecular Biology, pub. by Greene Publishing Assoc. and Wiley-lnterscience (1987).

### 1. Example 1: Fungal Isolates and Genomic DNA Extraction

Viable isolates of *Mycospaerella fijiensis* and *Erysiphe graminis* f. sp. *tritici* are obtained from Novartis Crop Protection, Stein, Switzerland. Further isolates of *Venturia inaequalis, Septoria tritici, Pseudocercosporella herpotrichoides, Fusarium culmorum, Botrytis cinerea,* and *Michrodochium nivale* are listed in Table 1 below.

**Table 1:**

| Source of Isolates | | | |
|---|---|---|---|
| Isolate | Species | Origin | Source |
| 184.97.1 | *Mycospaerella fijiensis* | Costa | CP Novartis |
| | | Rica | |
| 185.97.3 | *Mycospaerella fijiensis* | Costa | CP Novartis |
| | | Rica | |
| ----- | *Mycospaerella. Musae* | ----- | Novartis |
| Fel04 | *Erysiphe graminis* f. sp. *tritici* | Germany | CP Novartis |
| Fel08 | *Erysiphe graminis* f. sp. *tritici* | Germany | CP Novartis |
| Fel12 | *Erysiphe graminis* f. sp. *tritici* | Germany | CP Novartis |

The saprophytic fungi are grown in potato dextrose broth inoculated with mycelial fragments or spores from potato dextrose agar or from frozen stock cultures, respectively. Cultures are incubated on an shaker at 20 °C for 5-21 days. Mycelia are harvested by centrifugation, lyophilised and ground under liquid nitrogen. Total DNA extraction is performed after the protocol described in Sierotzki, H., M. Eggenschwiler, O. Boillat, J. M. McDermott, and C. Gessler (1994)' Detection of variation in virulence towards susceptible apple cultivars in natural populations of Venturia inaequalis'. Phytopathology **84**, 1005-1009.
The biotrophic fungus *E. graminis* is propagated on wheat plants (cv Kanzler) inoculated with conidia and incubated for 6-7 days at 20 °C and 100 % relative humidity. Conidia are harvested into a 1.5 ml Eppendorf tube and DNA extracted after the above method.

### 2. Example 2: Isolation of the cytochrome b gene

To clone and sequence the cytochrome b genes of *Mycosphaerella fijiensis,* a 200 bp long fragment is amplified with a touchdown program in a Perkin Elmer Cetus Gene Amp PCR System 9700 with degenerate primers (cyt-b1:5'-GAG CAY ATY ATG CGY GAY GT-3', cyt-b2rev: 5'-GT RAT RAC DAT RGC DCC CCA-3'; IUB code for mixed base sites). Amplification reaction volumes are 20 µl containing a reaction buffer (10 mM Tris-HCI pH 8.3; 50 mM KCI; 1.5 mM MgCl₂, Perkin Elmer), 100 µM each of dATP, dCTP, dGTP and dTTP (Boehringer, Mannheim, Germany), 0.3 µM Primer, 5 ng of genomic DNA and 1 U FAmpliTaq DNA polymerase (Perkin Elmer). The DNA is initially denatured for 5 min at 94 °C followed by 20 cycles with 1 min at 94 °C, 1 min at 50 °C and 1 min at 72 °C: in each cycle the annealing temperature decreases by 0.5 °C. Afterwards, 20 cycles are performed with annealing temperature at 40 °C with a final 5 min at 72 °C, then cooled down to 4 °C. Amplification products are electrophoresed in 1.5 % agarose (GibcoBRL, Rockville, MD) gels with 1 x TAE and stained with ethidium bromide. After cloning (TA cloning kit, Invitrogen) and sequencing (dRhodamin, ABI 377 Perkin Elmer) the sequences are analysed with AlFasta, BestFit, converted to amino acid sequences with GenBank yeast mitochondrial code (triplet tga codes for tryptophan) and compared with BLAST against NCBI/GenBank databases.
Specific primers are designed in forward (f) and reverse (r) direction (10f: 5'-GCA TTT AAT TCA GTA GAA CAC-3', 12f: 5'-CCC TGA GTA GGA CAA GAT ATA G-3' and 12r:
complement of 12f and 8r: 5'-CTA AAA AAT TTG CTA CGA AAA G-3'). These primers are used for RT-PCR in combination with the primer 5'-NN(T)₁₇-3'. Sequence comparisons between different isolates are based on at least two different PCR reactions.

To clone and sequence the cytochrome b genes of *Erysiphe graminis* a 200 bp long fragment has been amplified with a touchdown program in a Perkin Elmer Cetus Gene Amp PCR System 9700 with degenerate primers (cyt-b1:5'-GAG CAY ATY ATG CGY GAY GT-3', cyt-b2rev: 5'-GT RAT RAC DAT RGC DCC CCA-3'). The DNA is initially denatured for 3 min at 94 °C; followed by 20 cycles with 1 min at 94 °C, 1 min at 50 °C and 1 min at 72 °C. In each cycle the annealing temperature decreased by 0.5 °C. Afterwards another 20 cycles with annealing temperature at 40 °C are performed, followed by a final 5 min at 72 °C before cooling dwon to 4 °C. Amplification reaction volumes are 20 µl containing reaction buffer (10 mM Tris-HCI, pH 8.3, 50 mM KCl, 1.5 mM MgCl₂, Perkin Elmer), 125 µM each of dATP, dCTP, dGTP and dTTP (Boehringer, Mannheim, Germany), 0.5 µM Primer, 5 ng of genomic DNA and 0.625 U AmpliTaq DNA polymerase (Perkin Elmer). Amplification products are electrophoresed in 1.0 % agarose (GibcoBRL, Rockville, MD) gels with 1 x TAE and stained with ethidium bromide. After cloning (TA cloning kit, Invitrogen) and sequencing (dRhodamin, ABI 377 Perkin Elmer) the sequences are analysed with AlFasta, BestFit, converted to amino acid sequences with a GenBank general code (but triplet tga codes for tryptophan) and compared with BLAST against NCBI/GenBank databases. Specific primers have been designed (egcytb1f: 5'-GCG TGA TGT TAA TAA CGG-3', egcytb2f: 5'-CCT GGG TTA TGT ATT GCC-3' and egcytb2r: 5'-GGC AAT ACA TAA CCC AGG-3') to perform chromosome walking. Together with a new degenerate primer (cytbasco1: 5'-AAC ATN GCD ATN CAN CC-3') chromosome walking is used to generate a longer fragment of the gene. The specific primers, egcytb1f and egcytb4r: 5'-GGT ATA GAT CTT AAT ATT GC-3', are used for sequencing. Amplification with specific primers are performed with the reaction conditions as for degenerate primers, but with a three temperature program (annealing temperature of 50 °C for 1 min).

In both pathogens, about 100 bases of the cytochrome b gene in the 5' direction are not yet sequenced.
Sequence comparisons of a resistant and a sensitive isolate of both pathogens, reveale a difference in the nucleotide sequence (Figure 1, 2, respectively). This leads to the change of alanine to glycine in deduced amino acid sequences in both pathogens.

### 3. Example 3: Synthesis and Purification of Oligonucleotides

Oligonucleotides (primers) are synthesised by Microsynth GmbH, Schützenstrasse 15, 9436 Balgach, Switzerland.

### 4. Example 4: Polymerase Chain Reaction Amplification

Polymerase chain reactions are performed with the polymerase rTaq from Pharmacia using GeneAmp 10X Buffer and MgCl2 from Perkin Elmer (50 mM KCI, 2.5 mM MgCl₂, 10 mM Tris-HCI, pH8.3, containing 125 µM of each TTP, dATP, dCTP, and dGTP, 50 pM primer, 0.625 units of *Taq* polymerase and 10 ng of genomic DNA in a final volume of 20 µl. Reactions are run for 30 cycles of 60 s at 94°C, 60 s at 50°C, 60°C or 70°C, and 60 s at 72°C in a Perkin-Elmer/Cetus Model 9600 or 9700 thermal cycler. The products are analyzed by loading 10 µl of each PCR sample on a 1.0 % agarose gel and electrophoresing.

### 5. Example 5: DNA Extraction from leaf tissue

The bulk maceration method is used to isolate DNA from several naturally infected wheat leaves to optimize the field sampling method for high throughput analysis.

Bulk Maceration Method:
(1) Place the appropriate number of wheat heads or stems in a Bioreba (Reinach, Switzerland) heavy duty plastic bag (cat#490100). Weigh the plant tissue, plastic bag with leaves minus the tare (weight of the plastic bag).
(2) Add an equal volume (ml) of Muller Extraction Buffer (0.1% w/v Tween-80; 0.04 M Tris-CI, pH 7.7; 0.15 M NaCI; 0.1% w/v BSA-Pentex fraction V; 0.01% w/v sodium azide; 200 mM EDTA) per weight (g) of wheat tissue.
   Macerate the tissue using a Bioreba Homex 6 homogenizer set at 70. Grind the leaves until the tissue is fibrous.
(3) Pool the extracts from multiple bags, if used, and vortex well. Aliquot the extraction juice into eppendorf tubes on ice.
   (a) Boil 100 µl of the concentrated extract for 5 minutes.
   (b) Place the boiled extract on ice.
   (c) Make a 1:10 dilution by adding 10 µl from the boiled, concentrated extract to 90 µl of sterile dH₂O.
Store the diluted extracts on ice until ready to use.

**Table 2:**

| Source of Isolates | | | |
|---|---|---|---|
| Isolate | Species | Origin | Source |
| 184.97.1 | *Mycospaerella fijiensis* | Costa | CP Novartis |
| | | Rica | |
| 185.97.3 | *Mycospaerella fijiensis* | Costa | CP Novartis |
| | | Rica | |
| ----- | *Mycospaerella. Musae* | ----- | Novartis |
| Fel04 | *Erysiphe graminis* f. sp. *tritici* | Germany | CP Novartis |
| Fel08 | *Erysiphe graminis* f. sp. *tritici* | Germany | CP Novartis |
| Fel12 | *Erysiphe graminis f.* sp. *tritici* | Germany | CP Novartis |
| ------ | *Erysiphe graminis f.* sp. *hordeii* | ----- | Novarts |
| R-5126 | *F. culmorum* | Minnesot | P. Nelson⁶ |
| | | a | |
| 18222 | *M. nivale* | Scotland | ATCC |
| 5.8. | *Venturia inaequalis* | Les | CP Novartis |
| | | Barges | |
| á | *Septoria tritici* | ----- | Novartis |
| ----- | *Botrytis cinerea* | ----- | Novartis |
| 308 | *P. herpotrichoides* R-type | ----- | Novartis |
| | | | Basel |

### 6. Example 6: Specificity of Primers

The primer pairs have to fulfil to following requirements for specificity: Primer pairs recognising sensitive or resistant isolates, as well as different species. Primers used for the particular pathogen are listed in Table 3.

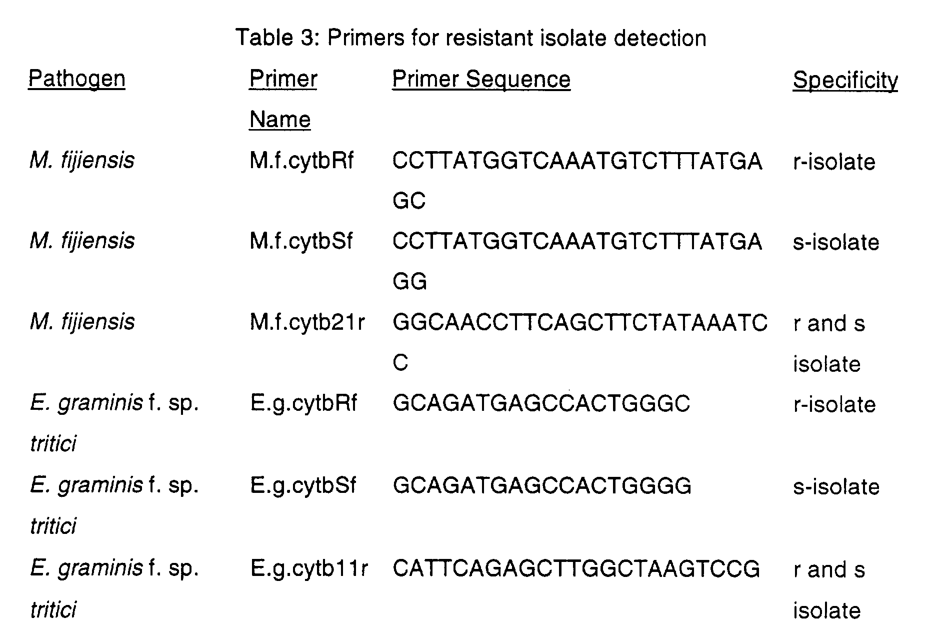

Among the pathogen species the primer pair specific for sensitivity differentiate in the last nucleotide at the 3'-prime end: g for sensitive and c for resistant. The species specificity is assured by selecting primers from regions unique to the particular species or by choosing primers of different parts of the gene.

### 7. Example 7: Determination of primers Specificity

PCR are performed according to Example 4 using the appropriate primer pairs to amplify the phenotype and species specific DNA fragments (Table 4).

**Table 4:**

| Specificity of primer pairs | | | | |
|---|---|---|---|---|
| (+ and - indicate presence and absence , respectively of the particular band) | | | | |
| Primer pair | *M. fijiensis* | | *E. graminis* f. sp. *tritici* | |
| | s-isolate | r-isolate | s-isolate | r-isolate |
| M.f.cytbSf/ | + | - | - | - |
| M.f.cytb21r | | | | |
| M.f.cytbRf/ | - | + | - | - |
| M.f.cytb21r | | | | |
| E.g.cytbSf/ | - | - | + | - |
| E.g.cytb11r | | | | |
| E.g.cytbRf/ | - | - | - | + |
| E.g.cytb11r | | | | |

The species specificity is tested using genomic DNA from various fungi, including pathogen from the same ecological niche. The primer pairs are also tested against extracts from the particular host. The species tested are listed in Table 1. The primer pair for the non-mutated DNA of *E. graminis* f. sp. *tritici* amplifies the particular DNA fragment in *E. graminis* f. sp. *hordei.* Similarly, the primer pair for the non-mutated DNA of *M. fijiensis* amplifies the particular DNA fragment in *M. musae.* All other fungal DNA give no amplification product. The primer combinations amplify no products from extracts of healthy wheat or banana plants.

## Claims

1. An oligonucleotide which is sufficiently similar to a region of the cytochrome b gene which is important for ligand binding such as, for example the region corresponding to amino acid position 127-147 and 275-296 respectively, to anneal to said region.

2. An oligonucleotide according to claim 1, wherein said region corresponds to amino acid position 143.

3. An oligonucleotide according to claim 1 or 2, wherein said cytochrome b gene is a gene selected from the group consisting of SEQ ID NO:7 and SEQ ID NO:11.

4. An oligonucleotide according to any one of claims 1 to 3, wherein said cytochrome b gene is resistant to a plant fungal pathogen based on at least one point mutation in the native gene.

5. An oligonucleotide according to claim 4, wherein said cytochrome b gene is a gene selected from the group consisting of SEQ ID NO:8, SEQ ID NO:9 and SEQ ID NO:10.

6. An oligonucleotide according to any one of claims 1 to 3 which has sequence identity with at least about 5-10 nucleotides comprised in the oligonucleotide sequence selected from the group consisting of SEQ ID NO: 1, SEQ ID NO: 2, SEQ ID NO: 3 SEQ ID NO: 4 SEQ ID NO: 5 SEQ ID NO: 6.

7. An oligonucleotide selected from the group consisting of SEQ ID NO: 1, SEQ ID NO: 2, SEQ ID NO: 3 SEQ ID NO: 4 SEQ ID NO: 5 SEQ ID NO: 6.

8. An oligonucleotide primer comprising all or part of the previously claimed oligonucleotides.

9. An oligonucleotide primer according to claim 8 which primer is an allele-specific primer and sufficiently similar to a region of a mutant cytochrome b gene which is resistant to a plant fungal pathogen based on at least one point mutation in the native gene, wherein said point mutation *is preferably located in a region at the 3'- end comprising between 1 to 5, preferably between 1 to 3 and most preferably 1 nucleotide.*

10. An oligonucleotide primer which is a species-specific primer capable of annealing *to* regions of the cytochrome b gene that are unique to the particular species and in a suitable distance apart from the region annealing with the allele-specific primers *for* PCR amplification, which distance comprises about 100 to about 10'000, preferably about 100 to about 4000 and most preferably about 100 to about 2000 base pairs

11. An oligonucleotide primer according to claim 10, wherein the fungal species are fungal pathogens selected from the group consisting of *Erysiphe graminis* and *Mycosphaerella fijiensis.*

12. A pair of oligonucleotide primers for use in the amplification-based detection of a *M. fijiensis* oligonucleotide DNA sequence selected from the group consisting of SEQ ID NO: 2 and SEQ ID NO: 3, SEQ ID NO: 1 and SEQ ID NO: 3.

13. A pair of oligonucleotide primers for use in the amplification-based detection of a *E. graminis* f. sp. *tritici* oligonucleotide DNA sequence selected from the group consisting of SEQ ID NO: 5 and SEQ ID NO: 6, SEQ ID NO: 4 and SEQ ID NO: 6.

14. A method for the detection of resistant isolates of a pathogen that is resistant to a fungicide targeting the Qo center of the cytochrome b gene such as, for example, the STAR fungicides comprising:
(a) isolating DNA from a plant, preferably a plant leaf, infected with a pathogen;
(b) subjecting said DNA to polymerase chain reaction amplification using
(i) an allele-specific primer recognising DNA of resistant isolates in combination with
(ii) a species-specific primer,
wherein, in case of a point mutation, the allele-specific primer differentiate in at least one nucleotide at the 3'- end of the respective primer, which nucleotide is preferably located in a region at the 3'- end comprising between 1 to 5, preferably between 1 to 3 and most preferably 1 nucleotide
and the species specific primer anneals to DNA sequences which are identical within a given species and differential among species
perferably in a region which is about 100 to about 10'000, preferably about 100 to about 4000 and most preferably about 100 to about 2000 base pairs apart from the region annealing with the allele-specific primer,
(c) detecting said fungal pathogen by visualizing the product or products of said polymerase chain reaction amplification.

15. A method for distinguishing between resistant and sensitive isolates of a plant fungal pathogen, preferably a pathogen that is resistant to a fungicide targeting the Qo center of the cytochrome b gene such as, for example, the STAR fungicides comprising the steps of:
(a) isolating DNA from a plant, preferably a plant leaf, infected with a pathogen;
(b) subjecting said DNA to polymerase chain reaction amplification using
(I) an allele-specific primer recognising DNA of sensitive isolates in combination with a species-specific primer,
(ii) an allele-specific primer recognising DNA of resistant isolates in combination with a species-specific primer,
wherein, in case of a point mutation, the allele-specific primers differentiate in at least one nucleotide at the 3'- end of the respective primer, which nucleotide is preferably located in a region at the 3'-end comprising between 1 to 5, preferably between 1 to 3 and most preferably 1 nucleotide
and the species specific primer anneals to DNA sequences which are identical within a given species and differential among species perferably in a region which is about 100 to about 10'000, preferably about 100 to about 4000 and most preferably about 100 to about 2000 base pairs apart from the region annealing with the allele-specific primer and
(c) detecting said fungal pathogen by visualizing the product or products of said polymerase chain reaction amplification.

16. A method for the detection of frequency of resistant isolates of a fungal pathogen, preferably a pathogen that is resistant to a fungicide targeting the Qo center of the cytochrome b gene such as, for example, the STAR fungicides comprising the steps of:
(a) isolating DNA from a plant, preferably a plant leaf, infected with a pathogen;
(b) subjecting said DNA to polymerase chain reaction amplification using
(I) an allele-specific primer recognising DNA of sensitive isolates in combination with a species-specific primer,
(ii) an allele-specific primer recognising DNA of resistant isolates in combination with a species-specific primer,
wherein, in case of a point mutation, the allele-specific primers differentiate in at least one nucleotide at the 3'- end of the respective primer, which nucleotide is preferably located in a region at the 3'-end comprising between 1 to 5, preferably between 1 to 3 and most preferably 1 nucleotide
and the species specific primer anneals to DNA sequences which are identical within a given species and differential among species perferably in a region which is about 100 to about 10'000, preferably about 100 to about 4000 and most preferably about 100 to about 2000 base pairs apart from the region annealing with the allele-specific primer and
(c) detecting said fungal pathogen by visualizing the product or products of said polymerase chain reaction amplification and.
(d) quantifiying the PCR products and calculating the ratio.

17. The method of any one of claims 14 to 16, wherein the sequence difference between the primers amplifying either a DNA fragment of sensitive or resistant isolates is at the 3' end of the primers recognizing the point mutation at amino acid position 143.

18. The method any one of claims 14 to 17, wherein said fungal pathogen is from *Erysiphe graminis* DC. f. sp. *tritici.*

19. The method any one of claims 14 to 17, wherein said fungal pathogen is from *Mycosphaerella fijiensis.*

20. The method of claims 18, wherein the allele-specific primer recognising DNA of sensitive isolates is the primer of SEQ ID NO: 5, the allele-specific primer recognising DNA of resistant isolates is SEQ ID NO: 4 and the reverse primer specific for *Erysiphe graminis* DC. f. sp. *tritici.*
is the primer of SEQ ID NO: 6.

21. The method of claims 18, wherein the allele-specific primer recognising DNA of sensitive isolates is the primer of SEQ ID NO: 2, the allele-specific primer recognising DNA of resistant isolates is SEQ ID NO: 1 and the reverse primer specific for *Mycosphaerella fijiensis* is the primer of SEQ ID NO: 3.

22. A kit for the detection resistant isolates comprising unlabeled or detectably labeled DNA primers according any one of claims 9 to 13.
